(19) Europäisches Patentamt
European Patent Office
Office européen des brevets

(11) EP 1 595 146 B1

(12) **EUROPEAN PATENT SPECIFICATION**

(45) Date of publication and mention of the grant of the patent:
**23.04.2008 Bulletin 2008/17**

(21) Application number: **04710922.8**

(22) Date of filing: **13.02.2004**

(51) Int Cl.:
***G01N 33/94*** *(2006.01)*

(86) International application number:
**PCT/GB2004/000571**

(87) International publication number:
**WO 2004/074844 (02.09.2004 Gazette 2004/36)**

(54) **SCREENING ASSAYS FOR CANNABINOID-LIGAND-TYPE MODULATORS OF GPR55**

SCREENING-TESTS FÜR CANNABINOID-LIGAND-ARTIGE MODULATOREN VON GPR55

DOSAGES DE CRIBLAGE POUR MODULATEURS DE TYPE LIGANDS CANNABINOIDES DE GPR55

(84) Designated Contracting States:
**AT BE BG CH CY CZ DE DK EE ES FI FR GB GR HU IE IT LI LU MC NL PT RO SE SI SK TR**

(30) Priority: **18.02.2003 US 448665 P**

(43) Date of publication of application:
**16.11.2005 Bulletin 2005/46**

(73) Proprietor: **AstraZeneca AB**
**151 85 Södertälje (SE)**

(72) Inventors:
- **DRMOTA, Tomas**
  **S-431 83 Molndal (SE)**
- **GREASLEY, Peter**
  **S-431 83 Molndal (SE)**
- **GROBLEWSKI, Thierry,**
  **Astrazeneca R & D Montreal**
  **St Laurent, Québec H4S 1Z9 (CA)**

(56) References cited:
**WO-A-01/86305**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 595 146 B1

**SEQUENCE LISTING**

<110> AstraZeneca AB

<120> Novel Assays

<130> LDG/100944-1

<150> US 60/448,665
<151> 2003-02-18

<160> 9

<170> PatentIn version 3.2

<210> 1
<211> 319
<212> PRT
<213> Homo sapiens

<400> 1

```
Met Ser Gln Gln Asn Thr Ser Gly Asp Cys Leu Phe Asp Gly Val Asn
1               5                   10                  15

Glu Leu Met Lys Thr Leu Gln Phe Ala Val His Ile Pro Thr Phe Val
            20                  25                  30

Leu Gly Leu Leu Leu Asn Leu Leu Ala Ile His Gly Phe Ser Thr Phe
        35                  40                  45

Leu Lys Asn Arg Trp Pro Asp Tyr Ala Ala Thr Ser Ile Tyr Met Ile
    50                  55                  60

Asn Leu Ala Val Phe Asp Leu Leu Leu Val Leu Ser Leu Pro Phe Lys
65                  70                  75                  80

Met Val Leu Ser Gln Val Gln Ser Pro Phe Pro Ser Leu Cys Thr Leu
                85                  90                  95

Val Glu Cys Leu Tyr Phe Val Ser Met Tyr Gly Ser Val Phe Thr Ile
            100                 105                 110

Cys Phe Ile Ser Met Asp Arg Phe Leu Ala Ile Arg Tyr Pro Leu Leu
        115                 120                 125

Val Ser His Leu Arg Ser Pro Arg Lys Ile Phe Gly Ile Cys Cys Thr
    130                 135                 140

Ile Trp Val Leu Val Trp Thr Gly Ser Ile Pro Ile Tyr Ser Phe His
145                 150                 155                 160

Gly Lys Val Glu Lys Tyr Met Cys Phe His Asn Met Ser Asp Asp Thr
                165                 170                 175

Trp Ser Ala Lys Val Phe Phe Pro Leu Glu Val Phe Gly Phe Leu Leu
            180                 185                 190
```

Pro Met Gly Ile Met Gly Phe Cys Cys Ser Arg Ser Ile His Ile Leu
195 200 205'

Leu Gly Arg Arg Asp His Thr Gln Asp Trp Val Gln Gln Lys Ala Cys
210 215 220

Ile Tyr Ser Ile Ala Ala Ser Leu Ala Val Phe Val Val Ser Phe Leu
225 230 235 240

Pro Val His Leu Gly Phe Phe Leu Gln Phe Leu Val Arg Asn Ser Phe
245 250 255

Ile Val Glu Cys Arg Ala Lys Gln Ser Ile Ser Phe Phe Leu Gln Leu
260 265 270

Ser Met Cys Phe Ser Asn Val Asn Cys Cys Leu Asp Val Phe Cys Tyr
275 280 285

Tyr Phe Val Ile Lys Glu Phe Arg Met Asn Ile Arg Ala His Arg Pro
290 295 300

Ser Arg Val Gln Leu Val Leu Gln Asp Thr Thr Ile Ser Arg Gly
305 310 315

<210> 2
<211> 319
<212> PRT
<213> Homo sapiens

<400> 2

```
Met Ser Gln Gln Asn Thr Ser Gly Asp Cys Leu Phe Asp Gly Val Asn
1               5                   10                  15

Glu Leu Met Lys Thr Leu Gln Phe Ala Val His Ile Pro Thr Phe Val
            20                  25                  30

Leu Gly Leu Leu Leu Asn Leu Leu Ala Ile His Gly Phe Ser Thr Phe
        35                  40                  45

Leu Lys Asn Arg Trp Pro Asp Tyr Ala Ala Thr Ser Ile Tyr Met Ile
    50                  55                  60

Asn Leu Ala Val Phe Asp Leu Leu Leu Val Leu Ser Leu Pro Phe Lys
65                  70                  75                  80

Met Val Leu Ser Gln Val Gln Ser Pro Phe Pro Ser Leu Cys Thr Leu
                85                  90                  95

Val Glu Cys Leu Tyr Phe Val Ser Met Tyr Gly Ser Val Phe Thr Ile
            100                 105                 110
```

Cys Phe Ile Ser Met Asp Arg Phe Leu Ala Ile Arg Tyr Pro Leu Leu
115 120 125

Val Ser His Ser Gly Pro Pro Gly Arg Ser Leu Gly Ser Ala Cys Thr
130 135 140

Ile Trp Val Leu Val Trp Thr Gly Ser Ile Pro Ile Tyr Ser Phe His
145 150 155 160

Gly Lys Val Glu Lys Tyr Met Cys Phe His Asn Met Ser Asp Asp Thr
165 170 175

Trp Ser Ala Lys Val Phe Phe Pro Leu Glu Val Phe Gly Phe Leu Leu
180 185 190

Pro Met Gly Ile Met Gly Phe Cys Cys Ser Arg Ser Ile His Ile Leu
195 200 205

Leu Gly Arg Arg Asp His Thr Gln Asp Trp Val Gln Gln Lys Ala Cys
210 215 220

Ile Tyr Ser Ile Ala Ala Ser Leu Ala Val Phe Val Val Ser Phe Leu
225 230 235 240

Pro Val His Leu Gly Phe Phe Leu Gln Phe Leu Val Arg Asn Ser Phe
245 250 255

Ile Val Glu Cys Arg Ala Lys Gln Ser Ile Ser Phe Phe Leu Gln Leu
260 265 270

Ser Met Cys Phe Ser Asn Val Asn Cys Cys Leu Asp Val Phe Cys Tyr
275 280 285

Tyr Phe Val Ile Lys Glu Phe Arg Met Asn Ile Arg Ala His Arg Pro
290 295 300

Ser Arg Val Gln Leu Val Leu Gln Asp Thr Thr Ile Ser Arg Gly
305 310 315

<210> 3
<211> 27
<212> DNA
<213> Artificial

<220>
<223> Oligonucleotide Primer

<400> 3
atgagtcagc aaaacaccag tggggac 27

<210> 4

<211> 24
<212> DNA
<213> Artificial

<220>
<223> Oligonucleotide Primer

<400> 4
ttagccccgg gagatcgtgg tgtc 24

<210> 5
<211> 69
<212> DNA
<213> Artificial

<220>
<223> Oligonucleotide Primer

<400> 5

**caagctagct atgccaccat ggactacaag gacgacgatg acaagagtca gcaaaacacc 60**

**agtggggac 69**

<210> 6
<211> 36
<212> DNA
<213> Artificial

<220>
<223> Oligonucleotide Primer

<400> 6
cctggatcca aattagcccc gggagatcgt ggtgtc 36

<210> 7
<211> 11
<212> PRT
<213> Homo sapiens

<400> 7

**Leu Arg Ser Pro Arg Lys Ile Phe Gly Ile Cys**
**1 5 10**

<210> 8
<211> 11
<212> PRT
<213> Homo sapiens

<400> 8

**Ser Gly Pro Pro Gly Arg Ser Leu Gly Ser Ala**
**1 5 10**

<210> 9
<211> 960
<212> DNA
<213> Homo sapiens

<400> 9

```
atgagtcagc aaaacaccag tggggactgc ctgtttgacg gtgtcaacga gctgatgaaa     60
accctacagt ttgcagtcca catccccacc ttcgtcctgg gcctgctcct caacctgctg    120
gccatccatg gcttcagcac cttccttaag aacaggtggc ccgattatgc tgccacctcc    180

atctacatga tcaacctggc agtctttgac ctgctgctgg tgctctccct cccattcaag    240
atggtcctgt cccaggtaca gtccccttc ccgtccctgt gcaccctggt ggagtgcctt     300
tacttcgtca gcatgtacgg aagcgtcttc accatctgct tcatcagcat ggaccggttc    360
ttggccatcc gttacccgct actggtgagc cactccggtc ccccaggaag atctttggga    420
tctgcatgca caatctgggt cctggtgtgg accggaagca tccctatcta cagtttccat    480
gggaaagtgg aaaaatacat gtgcttccac aacatgtctg atgatacctg gagcgccaag    540
gtcttcttcc cgctggaggt gtttggcttc ctccttccca tgggcatcat gggcttctgc    600
tgctccagga gcatccacat cctgctgggc cgccgagacc acacccagga ctgggtgcag    660
cagaaagcct gcatctacag catcgcagcc agcctggctg tattcgtggt ctccttcctc    720
ccagtccacc tggggttctt cctgcagttc ctggtgagaa acagctttat cgtagagtgc    780
agagccaagc agagcatcag cttcttcttg caattgtcca tgtgtttctc caatgtcaac    840
tgctgcctgg atgttttctg ctactacttt gtcatcaaag aattccgcat gaacatcagg    900
gcccaccggc cttccagggt ccagctggtc ctgcaggaca ccacgatctc ccggggctaa    960
```

## Description

### FIELD OF THE INVENTION

The present invention relates to screening assays for compounds that modulate the interaction between cannabinoids and other ligands and the GPR55 receptor and other cannabinoid receptors.

### BACKGROUND

Preparations of *Cannabis sativa* have been used for medicinal and recreational purposes for at least 4,000 years. Recently, cannabinoids have been the subject of renewed interest for their potential medicinal applications, *e.g.,* in analgesia, nausea and appetite stimulation.

Cannabanoids exert their effects by binding to specific receptors located in the cell membrane. Two types of high-affinity cannabinoid receptors have been identified to date by molecular cloning: 1) CB1 receptors (Devane et al., 1988, Mol. PharmacoL, 34:605-613; Matsuda et al., 1990, Nature, 346:561-564; Shire et al., 1995, J. BioL Chem, 270: 3726-3731; Ishac et al., 1996, Br. J. Pharmacol., 118:2023-2028), and 2) CB2 receptors (Munro et al., 1993, Nature, 365:61-65). Both CB1 and CB2 are coupled to the $G_i$, G-protein signal transduction pathway. Activation of the cannabinoid receptors leads to inhibition of adenylate cyclase and activation of MAP kinase. CB1 receptors can also modulate ion channels, inhibiting N-, and P/R-type calcium channels, stimulating inwardly rectifying $K^+$ channels and enhancing the activation of the A-type $K^+$ channel.

CB1 receptors are primarily, but not exclusively, expressed in the CNS and are believed to mediate the CNS effects of endogenous (*e.g.,* anandamide) and exogenously applied cannabinoids. Peripheral areas of expression include, but are not restricted to, the pituitary gland, immune cells, reproductive tissues, gastrointestinal tissues, superior cervical ganglion, heart, lung, urinary bladder, and adrenal gland. CB1 receptors are also located on central and peripheral nerve terminals and, when activated, seem to suppress the neuronal release of a number of excitatory and inhibitory transmitters including acetylcholine, noradrenaline, dopamine, 5 hydroxy-tryptamine, γ-aminobutyric acid, glutamate and aspartate (Pertwee, 1997, Pharmacol. Ther., 129:74; Ong & Mackie, 1999, Neuroscience, 92:1177; Pertwee, 2001, Progr. Neurobiol., 63:569). CB2 receptor expression is, however, restricted to the periphery, mainly in immune cells with particularly high levels in B-cells and natural killer cells (Galiègue et al., 1995, Eur. J. Biochem, 54:232).

A number of endogenous ligands have been identified which are believed to modulate the cannabinoid system via the previously identified CB1 and CB2 receptors, or by their action at as yet unidentified receptors. These ligands include, but are not limited to, anandamide, 2-aracadonylglycerol, noladin ether, palmitoylethanolamine, virohdamine and oleylthanolamide.

Endocannabinoids have been shown to play a role in the physiological regulation of food intake and body weight. Cannabinoid agonists are known to enhance appetite (Fride, 2002, Prostaglandins Leukot. Essent. Fatty Acids, 66:221-233). Studies have shown that anandamide increases food intake in rats, while the non-endogenous antagonist SR141716 (Sanofi Research) inhibits the in-take of palatable food.

Studies suggest that endocannabinoids regulate multiple physiological and pathological reproductive functions (Maccarrone et al., 2002, Prostaglandins Leukot. Essent. Fatty Acids, 66:309-317). The levels of uterine anandamide have been shown to affect pregnancy and embryo development in mice. High levels of anandamide in the mouse uterus have been associated with a decrease in uterine receptivity. Additionally, in women experiencing miscarriage, blood levels of anandamide were 4-fold higher than the levels in women with normal gestation.

Recent studies suggest that endocannabinoids, such as 2-arachidonoylglycerol, play a role in the progression of the pathophysiology of shock, since a selective CB1 antagonist reverses the hemorrhagic-induced shock condition (Cainazzo, 2002, Eur. J. Pharmacol, 441:91-97).

Endocannabinoids have also been shown to act as immunomodulators, generally exerting a negative action on the onset of a variety of parameters of the immune response (Parolaro et al., 2002, Prostaglandins Leukot. Essent. Fatty Acids, 66:319-32). Previous studies have shown that the CB2 receptor plays a very important role in the stimulation of growth of several, if not all, hematopoietic lineages (Valk et al., 1997, Blood, 90:1448-1457; Derocq, 2000, J. Biol. Chem., 275:15621-15628).

Thus, cannabinoid receptors are physiologically or pathophysiologically relevant in a great diversity of therapeutic areas, encompassing symptoms, disorders or syndromes of the central nervous, cardiovascular, endocrine or gastrointestinal systems. Specifically, the involvement of CB1 receptors has been implicated in altered pain perception, cognition and memory, addiction/substance abuse, schizophrenia/psychosis/delusion disorders, psychological, neurological, neurodegenerative (*i.e.,* Parkinson's disease), stress, mood modulation, depressive, anxiety, blood pressure regulation, gastrointestinal, eating and appetite disorders. Indeed, drugs known to interact with CB1 receptors are useful in the treatment or prevention of the aforementioned conditions.

In recent years a number of studies have suggested the existence of additional cannabinoid receptors. Calignano *et al.,* postulated the existence of an SR144528-sensitive non-CB1/CB2 cannabinoid receptor (1998, Nature, 394: 277-281). They presented evidence that although palmitoylethanolamide lacks significant affinity for CB1 or CB2 receptors, its ability to produce anti-hyperalgesia is readily attenuated by the CB antagonist/inverse agonist, SR144528. The presence of cannabinoid non-CB1/CB2 receptors in the mouse vas deferens has also been proposed. Evidence has also been obtained for the presence in vascular endothelium of an SR141716-sensitive non-CB1, non-CB2, non-vanilloid receptor that is unresponsive to established non-eicosanoid CB1/CB2 receptor agonists but can be activated by both eicosanoid cannabinoids, anandamide and methanandamide, as well as by certain classical cannabinoids ("abnormal cannabidiol "and its more potent analogue, O-1602) that do not act through CB or vanilloid receptors. Interestingly, two effects of abnormal cannabidiol, hypotension and mesenteric vasodilation, were found to be antagonized by the non-psychotropic classical cannabinoid, cannabidiol. So too was anandamide-induced mesenteric vasodilation. Finally, the existence in brain of non-CB1, non-CB2, SR141716-insensitive G protein-coupled receptors for anandamide has recently been proposed to explain results obtained from experiments with CB knockout mice.

In view of the role of CB1 and CB2 receptors in various diseases, there is a desire and need in the art to identify additional non-CB1 and -CB2 receptors that may also be implicated in disease and to use such receptors, alone or as part of a panel of other cannabinoid receptors, to identify and profile the effects of potential therapeutic compounds capable of treating one or other of the many diseases and disorders mediated by cannabinoid receptors.

G protein-coupled receptors (GPCRs) constitute a family of proteins sharing a common structural organization characterized by an extracellular N-terminal end, seven hydrophobic alpha helices putatively constituting transmembrane domains, and an intracellular C-terminal domain. GPCRs bind a wide variety of ligands that trigger intracellular signals through the activation of transducing G proteins (Caron et al., 1993, Rec. Prog. Horm. Res., 48:277-290; Freedman et al., 1996, Rec. Prog. Horm. Res., 51:319-353. More than 300 GPCRs have been cloned, and it is generally assumed than well over 1,000 of such receptors exist. Roughly 50-60% of all clinically relevant drugs act by modulating the functions of various GPCRs (Gudermann et al., 1995, J. Mol. Med., 73:51-63).

The cloning and sequence of the orphan GPCR GPR55 is disclosed in Sawzdargo et al. (Molecular Brain Research 64:193-198, 1999). The sequence is also disclosed in WO00/23588 and WO 01/86305. The cDNA sequence of wtGPR55 is disclosed in SEQ ID NO: 9. The encoded amino acid sequence is disclosed in SEQ ID NO:2.

## BRIEF DESCRIPTION OF THE DRAWINGS

Figure 1 shows the phylogenic relationship of CB1 and CB2 to GPR55.

Figure 2 shows the amino acid sequence of the human GPR55 variant, GPR55A (SEQ ID NO: 1), with a comparison to the sequence of GenBank accession number AF096786. The protein sequence for accession number AF096786 is distinct from GPR55A in that it contains the stretch of amino acids, SGPPGRSLGSA (SEQ ID NO:2), as shown in the figure. Also shown are the positions and changes of four different amino acid substitutions (# 4, 5, 10, and 12) that we have identified.

Figure 3 shows the specific binding of cannabinoid radioligands to membranes prepared from GPR55 and GPR55A transfected cells.

Figure 4 shows the concentration dependent displacement of [$^3$H]-SR141716 by SR141716 using membranes expressing CB1, GPR55 and GPR55A.

Figures 5A - 5D show GTPγS measurement of GPR55A activation in the presence of various concentrations of cannabinoid ligands.

Figure 6 shows concentration response curves for CP55940 on GPR55a expressed in HEK293s membranes treated with either pertussis toxin, cholera toxin or untreated. The concentration dependent activity remains unaltered whilst the background activity of the system is lowered.

## DETAILED DESCRIPTION

We have discovered that the orphan G protein-coupled receptor GPR55, and variant sequences thereof, binds and is activated by endogenous, natural and synthetic cannabinoid ligands. We have therefore discovered that GPR55 (and variant sequences thereof) is a novel cannabinoid receptor and a biological and pharmaceutical target of cannabinoid ligands. GPR55, as a member of class A of the G-protein coupled receptor family, possesses the classical motifs associated with this family, including seven putative hydrophobic transmembrane helices and a (E/D)R(YFS) sequence at the cytoplasmic end of transmembrane helix 3. Phylogenically, GPR55 it is poorly related to other receptors, showing closest identity (27.2%) to another orphan receptor, GPR35. Surprisingly, GPR55 has low sequence identity with the cannabinoid receptors CB1 (13.5%) and CB2 (14.4%).

The present invention is based upon our discovery that GPR55 displays high affinity for endogenous, natural and synthetic cannabinoid ligands and for ligands including, but not limited to, palmitoylethanolamine (PEA), a potent anti-inflammatory and anti-hyperalgesic molecule, and virohdamine. We have discovered that PEA and virohdamine, as well as anandamine, noladin ether, 2-aracadonylglycerol and oleylethanolamine, bind with high affinity to GPR55 and can activate this receptor. Pharmacological properties of these ligands can, therefore, be mediated by the modulation of GPR55 activity.

The interaction between cannabinoid and other ligands and the GPR55 receptor can be harnessed in a variety of assays to identify compounds that modulate the binding of cannabinoid and other ligands to GPR55, to identify compounds that modulate cannabinoid and other ligand activation of GPR55, to identify compounds that are agonists, antagonists, allosteric modulators, or inverse agonists of GPR55, and to identify compounds that selectively modulate GPR55, rather than other cannabinoid receptors such as CB1 or CB2. Assays of the present invention can also be used to identify compounds having activity at any combination of CB1, CB1a, CB1b, CB2 and GPR55.

Modulation of GPR55 activity by endogenous, natural or synthetic agonists, antagonists or inverse agonists may be useful for the treatment (therapeutic or prophylactic) of a number of diseases where cannabinoid ligands play a key role or have a beneficial effect, in particular but not limited to tissues where GPR55A is expressed and where cannabinoids are implicated to have a significant disease modifying effect, such as the prefrontal cortex, substantia nigra and nucleus basalis of Meynert in CNS and cognition disorders, eg. schizophrenia, Alzheimers disease and dementia, or the caudate and putamen in Parkinson's disease and depression. Also, in the heart and the cardiovascular system, the cannabinoids can have a disease modifying effect particularly through GPR55A on vascular tone and atherosclerotic lesions and also, but not limited to, myocardial infarction, congestive heart failure, coronary heart disease, hypertension, angina, stroke, arrythmia, peripheral vascular disease, renal disease and other vascular sequelaes of diabetes. Also, in the GI-tract the cannabinoids can have modulating effects through this receptor particularly on gastrointestinal motility related disorders such as IBS, IBD and GERD. Also cancers of GI-tract such as colon cancer, adenocarcinoma of the intestines and of the pancreas and liver in particular may be affected through cannabinoid modulation of this receptor. All forms of cancers of the CNS, in particular astrocytoma and glioblastoma and all forms of cancer of the lung, in particular adenocarcinomas, may be affected through cannabinoid modulation of this receptor. Compounds identified in using the assays of the present invention are furthermore useful for the treatment of obesity, e.g. by reduction of appetite and body weight, maintenance of weight reduction and prevention of rebound; psychiatric disorders such as psychotic disorders, e.g. schizophrenia and bipolar disorders, anxiety, anxio-depressive disorders, cognitive disorders, memory disorders, obsessive-compulsive disorders, anorexia, bulimia, attention disorders like ADHD, epilepsy, and related conditions; and, neurological disorders such as, multiple sclerosis, Raynaud's syndrome and Huntington's chorea. Such compounds are also potentially useful for the treatment of immune, cardiovascular, reproductive and endocrine disorders, septic shock and diseases related to the respiratory and gastrointestinal systems (e.g. diarrhea). Such compounds are also potentially useful as agents in treatment of extended abuse, addiction and/or relapse indications, e.g. treating drug (nicotine, ethanol, cocaine, opiates, etc) dependence and/or treating drug (nicotine, ethanol, cocaine, opiates, etc) withdrawal symptoms. Such compounds may also eliminate the increase in weight, which normally accompanies the cessation of smoking. Such diseases and disorders are herein referred to as cannabinoid receptor mediated diseases and disorders.

Particular examples of diseases and conditions that a modulatory compound against GPR55 may be used to treat include any of: hypertension, atherosclerosis and sequelas thereof, microvascular disease of Diabetes, Alzheimers disease, Parkinson disease, IBS, IBD, cancer of liver, cancer of pancreas and cancer of colon.

According to a first aspect of the invention there is provided a method for determining the ability of a test compound to modulate each of a panel of cannabinoid receptors, comprising measuring the modulatory effect of a test compound against the cannabinoid receptor having the amino acid sequence depicted in SEQ ID NO: 1 or a polypeptide with at least 90% sequence identity thereto, and at least one other cannabinoid receptor.

In one embodiment, the at least one other cannabinoid receptor is selected from the group consisting of: CB1, CB1a, CB1b and CB2.

In a further embodiment, a profile of the test compound is generated.

In a further embodiment, the receptor has the amino acid sequence depicted in SEQ ID NO: 1.

As used herein, the terms "modulate" or "modulates" in reference to binding include any measurable alteration to the binding interaction between a ligand and the GPR55 receptor, including, but not limited to, the amount or quantity of binding, binding affinity, and binding efficiency. For example, compounds identified using assays and methods of the present invention may increase or decrease the amount of binding of a ligand to the GPR55 receptor. Compounds identified using assays and methods of the present invention may enhance or inhibit the rate of binding of a ligand to the GPR55 receptor.

As used herein, the term "inhibit" in reference to binding of a ligand to the GPR55 receptor means any measurable decrease in binding.

As used herein, the term "decrease" in reference to cell stimulating activity or in reference to binding of a ligand to the GPR55 receptor means any measurable diminution of such cell stimulating activity or binding activity.

As used herein, the term "increase" in reference to cell stimulating activity or in reference to binding of a ligand to the GPR55 receptor means any measurable enhancement of such cell stimulating activity or binding activity.

As used herein, the terms "contact" or "contacting" refers to any method of combining components, such as combining compounds and/or a ligand in culture medium containing cells expressing the GPR55 receptor, or combining compounds and/or a ligand in solutions containing the GPR55 receptor, which may or may not be bound to a substrate.
As used herein, the phrase "functional fragment" in reference to the GPR55 receptor protein, refers to portions or fragments of the GPR55 receptor protein that are functionally active in the assays of the present invention, *i.e.,* are capable of binding to and/or being activated by a ligand (for example, a cannabinoid ligand). Functional fragment also includes fusion proteins that contain portions of the GPR55 receptor.
As used herein, the term "variant" in reference to the GPR55 receptor protein includes proteins having amino acid modifications, mutations, deletions, or insertions and other protein modifications that retain functionality in the assays of the present invention. Variant GPR55 receptor proteins include, but are not limited to, GPR55A, which is described further herein below. In one embodiment the variant possesses no more than 20, preferably no more than 10, and still more preferably no more than 5 amino acid substitutions from the sequence depicted in SEQ ID NO: 1 or 2.
One skilled in the art can readily determine whether a protein or peptide is a functional fragment of the GPR55 receptor by examining its sequence and testing for binding and/or activation activity without undue experimentation. For example, binding to a ligand known to bind GPR55, such as those identified in the examples herein, or the ability to activate $G\alpha_{13}$-protein. Truncated versions of the GPR55 receptor and fusion proteins containing portions of the GPR55 receptor may be prepared and tested using routine methods and readily available starting material. Typically, a polypeptide with at least 90%, at least 95%, at least 97% or at least 99% identity with the amino acid sequence of SEQID NO: 1, are considered as GPR55 variants. Variant polypeptides therefore include naturally occurring allelic variants as well as non-naturally occurring (or engineered) variants.
The sequence identity between two sequences can be determined by pair-wise computer alignment analysis, using programs such as, BestFit, Gap or FrameAlign. A commonly used alignment tool is BestFit. In practice, when searching for similar/identical sequences to the query search, from within a sequence database, it is generally necessary to perform an initial identification of similar sequences using suitable software such as Blast, Blast2, NCBI Blast2, WashU Blast2, FastA, Fasta3 and PILEUP, and a scoring matrix such as Blosum 62. Such software packages endeavour to closely approximate the "gold-standard" alignment algorithm of Smith-Waterman. Thus, the preferred software/search engine programme for use in assessing similarity, i.e. how two primary polypeptide sequences line up is Smith-Waterman. For the purpose of this invention the percent identity between two amino acid or nucleotide sequences is determined using the algorithm of E. Meyers and W. Miller (CABIOS, 4:11-17 (1989)) which has been incorporated into the ALIGN program (version 2.0), using a PAM120 weight residue table, a gap length penalty of 12 and a gap penalty of 4.
As used herein, the term "heterologous" in reference to the GPR55 receptor gene means any non-endogenous GPR55 receptor gene, for example, one that has been introduced or transfected into a cell, which includes GPR55 receptor genes from different species or organisms than the cell and recombinant GPR55 receptor genes from the same species or organism as the cell.
The present invention provides methods of identifying compounds that modulate the binding interaction of the GPR55 receptor or any variant or functional fragment thereof, including GPR55A, with a cannabinoid, or other ligand capable of binding to the GPR55 receptor. The binding interaction can be monitored by direct measurement of binding or by measurement of a signal indicative of receptor activation or cell stimulating activity.
Examples of signals that can be measured in assays of the present invention and which serve as indicators of receptor activation or indicators of cell stimulating activity include, but are not limited to, intracellular phospholipase C (PLC) activity, phospholipase A (PLA) activity, adenylyl cyclase activity, cAMP levels, MAP kinase activity, GTP$\gamma$S binding, and intracellular concentration of calcium in the cell, and opening and closing of ion channels.
Activation of the GPR55 receptor can be assayed by examination of a number of cellular signaling systems including, but not limited to, methods for measuring cAMP levels (alpha screen, reporter gene, scintillation proximity assays, etc.), methods for measuring intracellular calcium levels (FLIPR, use of promiscuous G-proteins such as $G_{16}$, $G_{qi5}$, $G_{qs5}$, etc.), methods for determining the activity of protein kinase A, protein kinase C, small G-protein activation, phospholipase C, small G-protein activation, and modulation of ion channel activity and function.
Many other methods of measuring receptor activation and cell stimulation are known to those skilled in the art and can be used in the assays of the present invention.
According to one embodiment of the invention, the modulatory effect of the test compound against each receptor is determined by:

(i) contacting said cells expressing the appropriate receptor with an appropriate ligand, in the presence or absence of a test compound;
(ii) determining the effect that the test compound has on the binding interaction of the receptor and the ligand; and
(iii) comparing the result in (ii) against each receptor in the panel.

According to particular aspects of the invention that involve determination of the binding interaction of a receptor and a ligand, the determination involves measuring the amount of cannabinoid or other ligand that forms a complex with the receptor, where an alteration to the amount of said complex formed in the presence of said test compound identifies said compound as a compound that modulates binding of the ligand to the receptor.

In one embodiment, the receptor/ligand complexes are isolated prior to measuring the amount of ligand in said complexes.

In other embodiments, the cannabinoid or other ligand is detectably labelled, for example, radiolabled, fluorescently labelled, or chemiluminescently labelled.

In another embodiment, the cannabinoid or other ligand is bound to an enzyme and measurement is carried out by enzyme-linked immunosorbent assay (ELISA).

In another embodiment, the receptor is provided as cells expressing the receptor or is provided as membranes prepared from said cells.

Any ligand that binds and/or activates the GPR55 receptor or variant or functional fragment or thereof can be used with the assays of the present invention. Such ligands include, but are not limited to cannabinoid ligands (such as endocannabinoids (endogenous), natural, and synthetic). As used herein, the terms "cannabinoid ligand" and "GPR55 ligand" are used interchangeably to refer to such compounds that bind to and/or interact with and/or activate the GPR55 receptor or variant or functional fragment thereof. Any endocannabinoid can be used with the methods of the present invention, including, but not limited to, anadamide, arachidonoyl glycerol, noladin ether, palmitoylethanolamine, and virodamine. Any plant-derived cannabinoid can be used with the methods of the present invention, including, but not limited to, Δ9-THC, cannabinol, and cannabidiol. Any plant-derived synthetic cannabinoid can be used with the methods of the present invention, including, but not limited to, CP55940, WIN55-212-2, HU210, AM251, AM281, AM630, JWH015, JWH133, ACEA, and ACPA. Radio-labelled cannabinoid receptor ligands that can be used with the methods of the present invention, include, but not limited to, [$^3$H]-SR141716, [$^3$H]-WIN55-212-2, [$^3$H]-CP55940, [$^3$H]-anandamide. Examples of suitable ligands for use in the screening methods of the invention include: anandamide, virohdamine, noladin ether, 2-arachidonoyl glycerol, palmityolethanolamine, oleylethanolamine and Δ9-tetrahydrocannabinol.

In some embodiments, cells expressing the GPR55 receptor are used in assays to identify compounds that modulate cannabinoid ligand/GPR55 binding. Cells expressing the GPR55 receptor can be incubated with a cannabinoid ligand and a test compound. Any cell type in which the GPR55 receptor is expressed, or can be engineered to be expressed, can be used. Any cell type in which receptor binding and/or receptor activation can be measured may be used in the assays of the invention. By way of non-limiting examples, the assay may utilize mammalian cells (including, but not limited to, human, hamster, mouse, rat, or monkey) or non-mammalian cells such as amphibian (*e.g.*, frog) or fish cells. Cell lines that may be used in the assays of the invention include, but are not limited to, HEK-293s (human embryonic kidney), CHO (Chinese hamster ovary), LTk- (murine fibroblasts lacking cytosolic deoxythymidine kinase (dTK)), HeLa, BALB/c-3T3, *Xenopus* oocytes; melanophores (cells from fish and amphibians) may also be used. In some embodiments, HEK-293s cells expressing the G protein $G_{\alpha 16}$ are used. Human cells are a preferred embodiment.

In some embodiments, a recombinant cell expressing a heterologous GPR55 receptor from a heterologous gene expression construct is used. Any species of GPR55 receptor may be used, including, but not limited to a mammalian GPR55 receptor, including human, rodent, murine, rat, guinea pig, mouse, hamster, rhesus, cynomologous monkey, and porcine. Human GPR55 is a preferred form. The GPR55 receptor protein may be a fusion protein or may have variation in amino acid sequence, including deletions, insertions, mutations, and polymorphisms.

Another aspect of the invention relates to methods of determining if a test compound is an agonist, antagonist, allosteric modulator, or inverse agonist of GPR55 receptor binding based upon a functional assay. In some embodiments, assays are carried out by incubating a cell expressing GPR55 receptor or a functional fragment or variant thereof with a test compound and determining whether intracellular phospholipase C, adenyl cyclase activity, cAMP levels, MAP kinase activity, GTPγS binding or intracellular calcium concentrations are modulated. Results can be compared with controls wherein incubations are performed in a similar manner but in the absence of the test compound. Functional assays of this type can be performed in conjunction with binding assays, including those described herein. In some embodiments, the cell used in functional assays is a recombinant cell that has been transformed with a heterologous GPR55 receptor gene or construct encoding GPR55 or functional fragment or variant thereof.

Test compounds that act as agonists are indicated by an increase in phospholipase C, a decrease or increase in adenylyl cyclase activity, a decrease or increase in cAMP levels, an increase in intracellular calcium levels, or an increase in the level of GTPγS binding.

Inverse agonists can reduce phospholipase C activity or intracellular calcium levels, particularly if assays are performed in the presence of a fixed amount of ligand. Antagonists block binding of ligand to the receptor but do not produce the opposite response in terms of phospholipase C activity, GTPγS binding levels, or intracellular calcium, that is the hallmark of an inverse agonist.

In some embodiments of the invention the GPR55 receptor is recombinantly expressed in cells from a heterogenous or heterologous gene. Nucleic acid sequences encoding the human GPR55 receptor can be cloned as described by Sawzdargo et al., 1999, Mol. Brain. Res., 64 193-198 (*see also,* GenBank accession number AF096786, WO 01/86305, and WO 00/23588, all incorporated herein by reference).

The GPR55 receptor coding sequence can be incorporated into an expression vector with a promoter and other regulatory elements that will be active and appropriate for expression in the particular cell type used (see, Sambrook et al., eds., Molecular Cloning: A Laboratory Manual (2nd ed.), Cold Spring Harbor Laboratory Press, Cold Spring Harbor, NY (1989)). In some embodiments, mammalian cells are used. Examples of promoters that may be used for expression in mammalian cells, include, but are not limited to, the mouse metallothionein I gene promoter (Hamer et al., 1982, J. Mol. Appl. Gen., 1:273-288), the immediate-early and TK promoter of herpes virus (Yao et al., 1995, J. Virol., 69: 6249-6258, McKnight, 1982, Cell, 31:355-365); the SV40 virus early promoter (Benoist et al., 1981, Nature, 290:304-310), and the CMV promoter (Boshart et al., 1985, Cell, 41:521-530). Vectors may also include enhancers and other regulatory elements.

Expression vectors can be introduced into cells by methods well known to the art, including, but not limited to, calcium phosphate precipitation, microinjection, electroporation, liposomal transfer, viral transfer, or particle-mediated gene transfer.

In some embodiments the GPR55 receptor is used to screen for compounds that mimic the action of cannabinoid or other GPR55 receptor ligands (agonists).

In some embodiments the GPR55 receptor is used to screen for compounds that antagonize the action of cannabinoid or other GPR55 receptor ligands (antagonists).

In some embodiments of any aspect of the invention, the human GPR55 receptor is used.

In some embodiments of any aspect of the invention, the GPR55A receptor is used.

Cells can be selected and assayed or examined for the expression of the GPR55 receptor according to standard procedures and techniques known to the art, including, but not limited to Northern blotting analysis.

In some embodiments, cells expressing the GPR55 receptor are used in conjunction with a cannabinoid ligand in screening assays designed to identify compounds that modulate cannabinoid ligand/GPR55 binding. Cells expressing the GPR55 receptor can be incubated with a cannabinoid ligand and a test compound. The extent to which the binding of the cannabinoid ligand is displaced by the test compound is then determined. Radioligand assays or enzyme-linked immunosorbent assays may be performed in which either the cannabinoid ligand or the test compound is detectably labelled. In certain embodiments, the cannabinoid or other ligand is radiolabelled, fluorescent labelled or chemiluminescent labelled.

In some embodiments, a cannabinoid or other ligand and cells expressing the GPR55 receptor are used in assays to determine whether test compounds have any modulatory effect on the binding between a cannabinoid or other ligand and the GPR55 receptor. A wide variety of different types of assays can be performed using standard methods known to those of skill in the art. For example, in radioligand binding assays, cells expressing the GPR55 receptor are incubated with a cannabinoid or other ligand and with a compound being tested for binding activity. In some embodiments, the source of the GPR55 receptor is recombinantly transformed HEK-293s cells. In some embodiments, other cells types are utilized, including cell types that do not express other proteins that bind the cannabinoid or other ligand being utilized. Such cell types can easily be determined by performing binding assays on cells transformed with GPR55 and comparing the results obtained with those obtained using their non-transformed counterparts.

In some embodiments of the invention, functional assays, such as mobilization of intracellular calcium, are carried out using a FLIPR (Fluorescent Imaging Plate Reader) detection system.

In some embodiments of the invention, the cannabinoid or other ligand is iodinated and used as a tracer in radioligand binding assays on whole cells or membranes. Other assays that can be used include, but are not limited to, the GTPγS assay, adenylyl cyclase assays, assays measuring inositol phosphates, and reporter gene assays (*e.g.,* those utilizing luciferase, aqueorin, alkaline phosphatase, etc.).

Assays may be performed using either intact cells or membranes prepared from the cells (see *e.g.,* Wang et al., Proc. Natl. Acad. Sci. U.S.A. 90:10230-10234 (1993)). In some embodiments, membranes or whole cells are incubated with a cannabinoid or other ligand and with a preparation of the compound being tested. After binding is complete, the receptor is separated from the solution containing the ligand and test compound, *e.g.,* by filtration, and the amount of binding that has occurred is determined. In some embodiments, the ligand used is detectably labelled with a radioisotope such as, for example, $^{125}I$. Other types of labels can also be used, including, but not limited to, the following fluorescent labeling compounds: fluorescein, isothiocynate, rhodamine, phycoerythrin, phycocyanin, allophycocyanin o-phthaldehyde and fluorescamine. Chemiluminescent compounds can also be used with the assays of the invention, including, but not limited to, luminol, isoluminol, theromatic of acridinium ester, imidazole, acridinium salt, and oxalate ester.

In some embodiments of the invention, assays are performed in a cell-free environment, such as, for example, where only the binding interaction between a cannabinoid or other ligand and the GPR55 receptor is being examined. In such cell-free or *in vitro* binding assays the cannabinoid or other ligand and/or the GPR55 receptor may be bound to a support.

In some embodiments of the invention, assays are carried out wherein the compound is tested at different concentrations and the signal measured at these different concentrations permits the binding affinity of the compounds to be determined.

Non-specific binding may be determined by carrying out the binding reaction in the presence of a large excess of unlabelled ligand. For example, labelled cannabinoid or other ligand may be incubated with GPR55 receptor and test compound in the presence of a thousand-fold excess of unlabelled cannabinoid ligand. Nonspecific binding can be subtracted from total binding, *i.e.*, binding in the absence of unlabelled ligand, to arrive at the specific binding for each sample tested. Other steps such as washing, stirring, shaking, filtering and the like can be included in the assays as necessary. Typically, wash steps are included after the separation of membrane-bound ligand from ligand remaining in solution and prior to quantitation of the amount of ligand bound, *e.g.*, by counting radioactive isotope. The specific binding obtained in the presence of test compound is compared with that obtained in the presence of labelled ligand alone to determine the extent to which the test compound has displaced receptor binding.

In performing binding assays, artifacts may falsely make it appear that a test compound is interacting with receptor when, in fact, binding is being inhibited by some other mechanism. Such artefact-generated false signals can be dealt with in a number of ways known to those of skill in the art. For example, the compound being tested can be placed in a buffer which does not itself substantially inhibit the binding of cannabinoid or other ligand to the GPR55 receptor, and compounds can be tested at several different concentrations. Preparations of test compounds can be examined for proteolytic activity and antiproteases can be included in assays. Additionally, compounds that are identified as displacing the binding of cannabinoid or other ligand to the GPR55 receptor can be re-examined in a concentration range sufficient to perform a Scatchard analysis on the results. This type of analysis is known to those of ordinary skill in the art and can be used for determining the affinity of a test compound for a receptor (see *e.g.,* Ausubel et al., eds., Current Protocols in Molecular Biology, 11.2.1-11.2.19, John Wiley & Sons, New York, NY (1993); Work et al., eds., Laboratory Techniques in Biochemistry and Molecular Biology, NY (1978)). Computer programs can be used to assist in the analysis of results (*e.g.,* Munson, 1983, Methods Enzymol., 92:543-577).

Depending upon their effect on the activity of the receptor, agents that inhibit the binding of the cannabinoid or other ligand to the GPR55 receptor may be either agonists or antagonists. Activation of the GPR55 receptor may be monitored using a number of different methods. For example, phospholipase C assays may be performed by growing cells in wells of a microtiter plate and then incubating the wells in the presence or absence of test compound total inositol phosphates (IP) may then be extracted in resin columns, and resuspended in assay buffer. Assay of IP thus recovered can be carried out using any method for determining IP concentration. Typically, phospholipase C assays are performed separately from binding assays, but it is also possible to perform binding and phospholipase C assays on a single preparation of cells.

Receptor activation can also be determined based upon a measurement of intracellular calcium concentration. Many types of assays for determining intracellular calcium concentrations are well known to the art and can be employed in the methods of the invention. For example, transformed HEK-293s can be grown to confluence on glass cover slides. After rinsing, the cells can be incubated in the presence of an agent such as Fluo-3, Fluo-4, or FURA-2 AM (Molecular Probes, Eugene, OR). After rinsing and further incubation, calcium displacement can be measured using a photometer.

Assays that measure the intrinsic activity of the receptor, such as those based upon inositol phosphate measurement, can be used to determine the activity of inverse agonists. Unlike antagonists that block the activity of agonists but produce no activity on their own, inverse agonists produce a biological response diametrically opposed to the response produced by an agonist. For example, if an agonist promoted an increase in intracellular calcium, an inverse agonist would decrease intracellular calcium levels.

The radioligand and cell activation assays described herein provide examples of the types of assays that can be used for determining whether a particular test compound alters the binding of the cannabinoid or other ligand to the GPR55 receptor and acts as an agonist or antagonist. There are many variations on these assays that are compatible with the present invention. Such assays can involve the use of labelled antibodies as a means for detecting the cannabinoid or other ligand that has bound to the GPR55 receptor or may take the form of the fluorescent imaging plate reader assays.

Another aspect of the present invention is directed to screening methods using GPR55 or any variant or functional fragment thereof, including GPR55A, in a matrix fashion with CB1 CB1a, CB1b and CB2, in order to identify compounds having discrete pharmacological profiles at these receptors. In some embodiments, the present invention provides assays to identify compounds that act selectively as agonists/antagonists/inverse agonists/allosteric modulators at only one of the cannabinoid receptors. In further embodiments, the present invention provides assays to identify compounds having activity at any two of these receptors. In still further embodiments, the present invention provides assays to identify compounds that act as agonists at one of the receptors but as antagonists at one of the others. Given the diverse roles of cannabinoids in biological processes, the assays of the present invention are useful for the identification and development of compounds with selective effect profiles. Compounds that selectively modulate a particular cannabinoid receptor may be particularly useful in treating distinct diseases mediated by the particular receptor without adversely affecting the normal physiology mediated by other receptors. In this way, compounds may be tailored to particular diseases and side-effects kept to a minimum.

According to another aspect of the invention there is provided a screening system wherein the modulatory ability of a test compound is determined by screening the compound against a panel of cannabinoid receptors, said panel comprising GPR55A receptor having the sequence depicted in SEQ ID NO: 1, or a variant thereof with at least 90% sequence identity to SEQ ID NO: 1, and at least one other cannabinoid receptor family member. Any of the screening methods disclosed herein could be used in this aspect of the invention.

In a particular embodiment, the "at least one other cannabinoid receptor family member" is selected from the group consisting of CB1, CB1a, CB1b and CB2.

According to a further aspect of the invention there is provided a method for determining the selectivity of a test compound against a cannabinoid receptor family member comprising determining the ability of the test compound to modulate each of a panel of cannabinoid receptors, said cannabinoid receptor panel comprising GPR55A receptor having the sequence depicted in SEQ ID NO: 1, or a variant thereof with at least 90% sequence identity to SEQ ID NO: 1, and at least one other cannabinoid receptor selected form CB1, CB1a, CB1b and CB2.

A profile of the effects of the test compound against each receptor can then be generated. Such profile may in its simplest form be a table of data depicting the modulatory effect of a test compound against each receptor.

According to a further aspect of the invention there is provided the use of GPR55A receptor having the sequence depicted in SEQ ID NO: 1, or a variant thereof with at least 90% sequence identity to SEQ ID NO: 1, and at least one other receptor selected from the group consisting of: CB1, CB1a, CB1b and CB2, in a panel screen testing the modulatory ability of a test compound against each of the receptors in the panel.

According to a further aspect of the invention there is provided the use of GPR55A receptor having the sequence depicted in SEQ ID NO: 1, or a variant thereof with at least 90% sequence identity to SEQ ID NO: 1, at least one other receptor selected from the group consisting of: CB1, CB1a, CB1b and CB2, and a cannabinoid ligand for binding to each receptor in a matrix assay to determine the modulatory effect of a test compound on binding of the ligand to the receptor.

Given the diverse roles of cannabinoids in biological processes, the assays of the present invention are useful for the identification and development of compounds with selective effect profiles. By selective, in respect of GPR55, we mean one exhibiting at least 10-fold more activity against GPR55 than either of CB1 or CB2.

The modulators identified according to the assays of the present invention are predicted to be of potential use in the treatment of certain of the cannabinoid receptor mediated diseases or disorders, such as those identified above.

The invention is further illustrated by way of the following examples, which are intended to elaborate several embodiments of the invention. These examples are not intended to, nor are they to be construed to, limit the scope of the invention.

## EXAMPLES

### Example 1. Cloning of GPR55A

In order to test whether GPR55 has any cannabinoid or cannabinoid like activity we carried out PCR and amplified and sequenced isolated cDNAs.

GPR55-specific primers were designed based on the human GPR55 sequence (EMBL-AF096786) and were used for PCR amplification of the entire GPR55 open reading frame. PCR was carried out using human genomic DNA (Clontech (Palo Alto, CA) cat#6550-1, lot#45641) as the template and GPR55F and GPR55R primers (shown below). The product generated in the PCR reaction was subsequently used as a template for the GPR55flagF and GPR55flagR primers in a second round of PCR to introduce a Flag epitope as well as NheI and BamHI sites. DNA fragments including the Flag epitope were then inserted into the corresponding restriction sites of the pIRESneo2 vector (Clontech, Palo Alto, CA).

**Primers**

| | |
|---|---|
| GPR55F | 5'-ATG AGT CAG CAA AAC ACC AGT GGG GAC-3' (SEQ ID NO:3) |
| GPR55R | 5'-TTA GCC CCG GGA GAT CGT GGT GTC-3' (SEQ ID NO:4) |

NheI Kozak Start Flag

GPR55flagF 5'-CAA GCT AGC TAT GCC ACC ATG GAC TAC AAG GAC GAC GAT GAC AAG AGT CAG CAA AAC ACC AGT GGG GAC-3' (SEQ ID NO:5)

BamHI Stop

GPR55flagR 5'-CCT GGA TCC AAA TTA GCC CCG GGA GAT CGT GGT GTC-3' (SEQ ID NO:6)

This led to the identification of an insertion and deletion within the published nucleotide sequence (Sawzdargo et al. 1999, Mol Brain Res., 64 193-198; GenBank accession number AF096786) that resulted in a change of 11 amino acids in the putative region of intracellular loop 2 and TM4.

The effect of insertion and deletion on GPR55 protein sequence is as follows:

| | | |
|---|---|---|
| GenBank AF096786: | ..S G P P G R S L G S A.. | GPR55 (SEQ ID NO:8) |
| Clone we obtained: | ..L R S P R K I F G I C.. | GPR55A (SEQ ID NO:7) |

The alignment of this change can be seen in Figure 2, which shows the complete protein sequence for GPR55A. This change, which we have named GPR55A, corresponds with the translation of the human genome sequence, and has also been identified in WO 00/23588. We have, however, also identified four other single nucleotide substitutions in our clones that result in single amino acid sequence substitution differences between GRP55A protein and the AF096786 protein sequence. These are also shown in Figure 1 and are represented by # designations (#4, 5, 10, and 12).

**Expression**

Expression of GPR55A is achieved by transient transfection of appropriate plasmid DNAs using HEK293s cells or another appropriate cell line and Lipofectamine Plus or alternative transfection reagent, according to the manufacture's recommendations. Cells are harvested and membranes prepared as previously described (Milligan, G. (1999) Signal Transduction: A Practical Approach, pages 65 - 67, Oxford University Press).

**Example 2. GRP55A has the ability to bind cannabinoid ligands when expressed in HEK293s cells.**

To test whether GPR55 and/or GPR55A encoded a receptor that had the potential to perform as a cannabinoid receptor we transiently transfected cDNAs encoding FLAG-tagged forms of the receptors into HEK293S cells using lipofectamine. The cells were harvested 48 hours later and membranes prepared.

**Membrane preparations**

Membrane preparations were prepared by re-suspending receptor expressing cells in ice cold TE buffer (10mM Tris-HCl, 0.1mM EDTA pH7.5) and leaving on ice for 5 minutes before pelleting the insoluble material by centrifugation at 1000g. This process was repeated twice before re-suspending the pellet in an appropriate volume of TE and storing at -80°C.

A FLAG-tagged CB1 receptor-expressing construct was included as a positive control for the experiment, whilst empty vector was included as a negative control.

In addition, transfections were performed for the purpose of immunohistochemistry to demonstrate that receptors were expressed at the cell surface (data not shown).

**Radioligand binding**

Test compounds were added followed by 1.6nM radioligand ($^3$H-SR-141716A, $^3$H -CP55940 or $^3$H -WIN-55,

212-2) to an assay mixture containing membranes (10μg protein) in 50mM Tris, 5.0mM $MgCl_2x6H_2O$ (pH 7.4) supplemented with 0.05% BSA. The reaction was allowed to proceed at 30°C for 90 min and then terminated by rapid filtration using GF/B filters (pre-soaked in 0.05% PEI) and washed with 50mM Tris (pH 7.4), $MgCl_2x6H_2O$. Filters were then covered with scintillant and counted for the amount of radio ligand retained. Non-specific binding was determined in the presence of 200nM SR-141716A, CP55940 or WIN-55, 212-2.

**Results**

We chose three cannabinoid radioligands ([$^3$H]-CP55940 - non selective cannabinoid agonist; [$^3$H]-WIN55-212-2 - non-selective cannabinoid agonist; [$^3$H]-SR141716, CB1 selective inverse agonist) as tools to test for specific binding in the GPR55 transfections using excess unlabelled of the same ligand to determine the level of non-specific binding. The results are shown in Figure 3.

No specific binding was obtained for un-transfected cells (not shown) or vector transfected negative control cells. However, specific binding was obtained as expected for the positive control CB1 transfected cells with each of the radioligands. Membranes prepared from cells transfected with either GPR55 or GPR55A displayed significant binding for [$^3$H]-CP55940 and [$^3$H]-SR141716, but little or no binding of [$^3$H]-WIN55-212, suggesting that GPR55 and GPR55A has some cannabinoid like ligand binding properties.

In order to further evaluate this binding, we generated competition-binding curves for [$^3$H]-SR141716 being competed by the unlabelled version of itself against GPR55, GPR55A, CB1 and vector control membranes. The results are shown in Figure 4. These data demonstrate an $IC_{50}$ of 0.3nM for SR141716 at CB1, as expected, and no specific binding to membranes derived from vector-transfected cells was observed. GPR55 and GPR55A both displayed nanomolar affinity displacement curves.

**Example 3. GPR55 and GRP55A have functional cannabinoid activities.**

To investigate whether GPR55 or GPR55A possessed any cannabinoid-like functional activity, receptor-expressing cell membranes were tested in a GTPγS assay. Membranes with or without 20μM ligand were assayed for their stimulatory or inhibitory activity on GTPγS binding. As a negative control, cell membranes were prepared from empty vector-transfected cells, and as a positive control, cell membranes were prepared from CB1 transfected cells.

**GTPγS binding assay**

Receptor activity was determined using a GTPγS binding assay as follows: 10μg of membranes diluted in 200μl of 100mM NaCl, 5mM $MgCl_2$, 1mM EDTA, 50mM HEPES (pH 7.4), 1mM DTT, 0.1% BSA and 100μM GDP. To this was added the required concentration of test compound and 0.1μCi $^{35}$S-GTPγS in the presence or absence of an $EC_{80}$ concentration of agonist (HU210). The reaction was allowed to proceed at 30°C for 45 min. Samples were then transferred on to GF/B filters using a cell harvester and washed with wash buffer (50mM Tris (pH 7.4), 5mM MgCl2, 50mM NaCl). Filters were then covered with scintilant and counted for the amount of $^{35}$S-GTPγS retained by the filter. To determine the level of non-specific binding control reactions were performed in the presence of 10μM GTPγS.

Functional activity of ligands either in the presence or absence of agonists was determined as follows: Non-specific binding was subtracted from all values determined. The effect of compounds at various concentrations was plotted according to the equation

$$y = A+((B-A)/1+((C/x)?\wedge D)))$$

and $EC_{50}$ or $IC_{50}$ estimated.

**Results**

No significant activity was detected for any compounds against membranes prepared from vector control transfections. The profile of compounds against CB1 was as expected for those ligands at CB1 receptors. Compounds acting at either GPR55 (data not shown) or GPR55A (see Table 1) displayed various effects upon GTPγS binding.

To further define the agonist activities of cannabinoid ligands $EC_{50}$ determinations were made. As before, none of the ligands displayed activity on control vector-transfected cells. Refer to Figures 5A - 5D for the results. CB1-transfected cells generated $EC_{50}$ values comparable to those expected for each ligand (data not shown). GPR55A displayed concentration-dependent activation in response to the natural ligands anandamide, Noladin ether, 2-arachidonoyl glycerol, palmityolethanolamine and Δ9-tetrahydrocannabinol. The data for these and other compounds are summarized in Table 1. **Table 1. Activity of ligands at GPR55A** (Antagonist data were generated in the presence of an $EC_{80}$ concentration of HU210.)

| **COMPOUND** | **$EC_{50}$ (nM) (except *)** | **n** |
|---|---|---|
| Anandamide | 18.4±4.1 | 6 |
| Noladin Ether | 11.5±0.5 | 2 |
| Palmitoylethanolamide | 3.2±1.3 | 3 |
| Virodhamine | 10.4±1.4 | 7 |
| 2-AG | 3.5±2.4 | 2 |
| Oleylethanolamide | 424±203 | 3 |
| Δ9-THC | 8.9±1.1 | 3 |
| CP55940 | 7.1±1.3 | 5 |
| Win55 | NA | 3 |
| Hu210 | 33±12 | 2 |
| JWH-015 | 4.75±0.2S | 2 |
| JWH-133 | NA | 2 |
| Cannabidiol | 354±63* | 4 |
| Cannabinol | NA | 3 |
| Abnormal cannabidiol | 2780±98 | 2 |
| NA= not active<br>* $IC_{50}$ measurement | | |

**Example 4 Assessing whether or not GPR55A couples to $G_i$ or $G_s$ G** proteins.

The use of pertussis and cholera toxins permits the selective inhibition of Gi and Gs G-protein activation. HEK293s cells transiently transfected with GPR55a were treated with either toxin and the membranes generated tested for their ability to generate a GTPγS signal. Figure 6 shows concentration response curves for CP55940 on GPR55a expressed in HEK293s membranes treated with either pertussis toxin, cholera toxin or untreated. The concentration dependent activity remains unaltered whilst the background activity of the system is lowered. Neither toxin was able to affect the GTPγS assay, demonstrating that GPR55a is coupled not to Gi or Gs in this system and therefore is probably coupled to another G-protein.

The foregoing examples are meant to illustrate the invention and are not to be construed to limit the invention in any way.

## Claims

1. A method for determining the ability of a test compound to modulate each of a panel of cannabinoid receptors, comprising measuring the modulatory effect of a test compound against the cannabinoid receptor having the amino acid sequence depicted in SEQ ID NO: 1 or a polypeptide with at least 90% sequence identity thereto, and at least one other cannabinoid receptor.

2. The method according to claim 1, wherein the at least one other cannabinoid receptor is selected from the group consisting of: CB1, CB1a, CB1b and CB2.

3. The method according to claim 1 or 2, further comprising generating a profile of the test compound.

4. The method according to any of the preceding claims, wherein the receptor has the amino acid sequence depicted in SEQ ID NO: 1.

5. The method according to any of the preceding claims, which method measures the effect that the test compound has on the binding interaction of the receptor and a ligand.

6. The method according to claim 5, wherein the binding interaction is monitored by direct measurement of binding or by measurement of a signal indicative of receptor activation or cell stimulating activity.

7. The method according to claim 6, wherein the signal indicative of receptor activation is selected from the group consisting of: intracellular phospholipase C activity, phospholipase A activity, adenylyl cyclase activity, cAMP level, MAP kinase activity, GTPγS binding, and intracellular concentration of calcium.

8. The method according to claim 6, wherein determining the binding interaction involves measuring the amount of ligand that forms a complex with the receptor, where an alteration in the amount of said complex formed in the presence of said test compound identifies said compound as a compound that modulates binding of the ligand to the receptor.

9. The method according to claim 8, wherein the ligand is selected from the group consisting of: anandamide, virohdamine, noladin ether, 2-arachidonoyl glycerol, palmityolethanolamine, oleylethanolamine and Δ9-tetrahydrocannabinol.

10. The method according to claim 8 or 9, wherein receptor/ligand complexes are isolated prior to measuring the amount of ligand in said complexes.

11. The method according to any of claims 5 to 10, wherein the ligand is detectably labelled.

12. The method according to claim 11, wherein the ligand is radiolabled, fluorescently labelled, or chemiluminescently labelled.

13. The method according to claim 8, wherein the ligand is bound to an enzyme, and measuring is carried out by enzyme-linked immunosorbent assay (ELISA).

14. The method according to any of the preceding claims, wherein the receptors are expressed from cells.

15. The method according to any of the preceding claims, wherein each receptor from the panel is expressed from a distinct populations of cells.

16. The method according to claims 14 or 15, wherein the cells are mammalian cells.

17. The method according to any of the preceding claims, wherein the receptor is expressed from a heterologous receptor gene.

18. The method according to any of the preceding claims, wherein the modulatory effect of the test compound against each receptor is determined by:

(i) contacting said cells expressing the appropriate receptor with an appropriate ligand, in the presence or absence of a test compound;
(ii) determining the effect that the test compound has on the binding interaction of the receptor and the ligand; and
(iii) comparing the result in (ii) against each receptor in the panel.

19. The use of GPR55A receptor having the sequence depicted in SEQ ID NO: 1, or a variant thereof with at least 90% sequence identity to SEQ ID NO: 1, and at least one other receptor selected from the group consisting of: CB1, CB1a, CB1b and CB2, in a panel screen to test the modulatory effect of a test compound against each of the receptors in the panel.

20. The use of GPR55A receptor having the sequence depicted in SEQ ID NO: 1, or a variant thereof with at least 90% sequence identity to SEQ ID NO: 1, at least one other receptor selected from the group consisting of: CB1, CB1a, CB1b and CB2, and a cannabinoid ligand for binding to each receptor in a matrix assay to determine the modulatory effect of a test compound on binding of the ligand to the receptor.

21. The use as claimed in claim 20, wherein the cannabinoid ligand is selected from the group consisting of: anandamide, virohdamine, noladin ether, 2-arachidonoyl glycerol, palmityolethanolamine, oleylethanolamine and Δ9-tetrahydrocannabinol.

**Patentansprüche**

1. Verfahren zur Bestimmung der Fähigkeit einer Testverbindung zur Modulation einer jeden Reihe von Cannabinoid-Rezeptoren, umfassend das Messen der modulatorischen Wirkung einer Testverbindung gegenüber dem Cannabinoid-Rezepor mit der in SEQ ID Nr. 1 angezeigten Aminosäuresequenz oder einem Polypeptid mit mindestens 90% Sequenzidentität dazu, und mindestens einem weiteren Cannabinoid-Rezeptor.

2. Verfahren nach Anspruch 1, wobei der mindestens eine weitere Cannabinoid-Rezeptor aus der aus CB1, CB1a, CB1b und CB2 bestehenden Gruppe ausgewählt ist.

3. Verfahren nach Anspruch 1 oder 2, zudem umfassend das Erzeugen eines Profils der Testverbindung.

4. Verfahren nach einem der vorhergehenden Ansprüche, wobei der Rezeptor die in SEQ ID Nr. 1 dargestellte Aminosäuresequenz hat.

5. Verfahren nach einem der vorhergehenden Ansprüche, wobei das Verfahren die Wirkung misst, die die Testverbindung auf die Bindungswechselwirkung des Rezeptors und eines Liganden hat.

6. Verfahren nach Anspruch 5, wobei die Bindungswechselwirkung durch direkte Messung der Bindung oder durch Messung eines Signals überwacht wird, das eine Rezeptoraktivierung oder Zellstimulierungsaktivität anzeigt.

7. Verfahren nach Anspruch 6, wobei das Signal, das die Rezeptoraktivierung anzeigt, aus der aus intrazellulärer Phospholipase-C-Aktivität, Phospholipase-A-Aktivität, Adenylylcyclaseaktivität, cAMP-Spiegel, MAP-Kinaseaktivität, GTP$\gamma$S-Bindung und intrazellulärer Calciumkonzentration bestehenden Gruppe ausgewählt ist.

8. Verfahren nach Anspruch 6, wobei die Bestimmung der Bindungswechselwirkung das Messen der Ligandenmenge umfasst, die einen Komplex mit dem Rezeptor bildet, wobei eine Veränderung der Menge des Komplexes, der sich in der Anwesenheit der Testverbindung gebildet hat, die Verbindung als eine Verbindung identifiziert, die die Bindung des Liganden an den Rezeptor moduliert.

9. Verfahren nach Anspruch 8, wobei der Ligand aus der aus Anandamid, Virohdamin, Noladinether, 2-Arachidonoylglycerin, Palmitoylethanolamin, Oleylethanolamin und D9-Tetrahydrocannabinol bestehenden Gruppe ausgewählt ist.

10. Verfahren nach Anspruch 8 oder 9, wobei die Komplexe aus Rezeptor und Ligand vor der Messung der Menge an Ligand in den Komplexen isoliert werden.

11. Verfahren nach einem der Ansprüche 5 bis 10, wobei der Ligand nachweisbar markiert ist.

12. Verfahren nach Anspruch 11, wobei der Ligand radioaktiv, fluoreszierend oder chemilumineszierend markiert ist.

13. Verfahren nach Anspruch 8, wobei der Ligand an ein Enzym gebunden ist, und die Messung durch Enzymimmuntest (ELISA) durchgeführt wird.

14. Verfahren nach einem der vorhergehenden Ansprüche, wobei die Rezeptoren aus Zellen exprimiert werden.

15. Verfahren nach einem der vorhergehenden Ansprüche, wobei jeder Rezeptor aus der Reihe von einer bestimmten Population von Zellen exprimiert wird.

16. Verfahren nach den Ansprüchen 14 oder 15, wobei die Zellen Säugetierzellen sind.

17. Verfahren nach einem der vorhergehenden Ansprüche, wobei der Rezeptor aus einem heterologen Rezeptorgen exprimiert wird.

18. Verfahren nach einem der vorhergehenden Ansprüche, wobei die modulatorische Wirkung der Testverbindung gegen jeden Rezeptor bestimmt wird durch:

(i) Zusammenbringen der Zellen, die den geeigneten Rezeptor exprimieren, mit einem geeigneten Liganden, in der Gegenwart oder Abwesenheit einer Testverbindung;
(ii) Bestimmung der Wirkung, die die Testverbindung auf die Bindungswechselwirkung des Rezeptors und des Liganden hat; und
(iii) Vergleichen des Ergebnisses in (ii) gegen jeden Rezeptor in der Reihe.

**19.** Verwendung des GPR55A-Rezeptors mit der in SEQ ID Nr. 1 dargestellten Sequenz oder einer Variante davon mit mindestens 90% Sequenzidentität zu SEQ ID Nr. 1, und mindestens eines anderen Rezeptors, ausgewählt aus der aus CB1, CB1a, CB1b und CB2 bestehenden Gruppe, in einem Reihentest zur Untersuchung der modulatorischen Wirkung einer Testverbindung gegen jeden der Rezeptoren in der Reihe.

**20.** Verwendung des GPR55A-Rezeptors mit der in SEQ ID Nr. 1 dargestellten Sequenz, oder einer Variante davon mit mindestens 90% Sequenzidentität zu SEQ ID Nr. 1, mindestens eines anderen Rezeptors, ausgewählt aus der aus CB1, CB1a, CB1b und CB2 bestehenden Gruppe und eines Cannabinoid-Liganden zur Bindung an jeden Rezeptor in einem Matrixtest zur Bestimmung der modulatorischen Wirkung einer Testverbindung auf die Bindung des Liganden an den Rezeptor.

**21.** Verwendung nach Anspruch 20, wobei der Cannabinoid-Ligand aus der aus Anandamid, Virohdamin, Noladinether, 2-Arachidonoylglycerin, Palmitoylethanolamin, Oleylethanolamin und D9-Tetrahydrocannabinol bestehenden Gruppe ausgewählt ist.

## Revendications

**1.** Procédé pour déterminer la faculté d'un composé de test à moduler chacun des récepteurs cannabinoïdes d'un panel, comprenant l'étape consistant à mesurer l'effet modulatoire d'un composé à tester vis-à-vis du récepteur cannabinoïde ayant la séquence d'acides aminés indiquée à la SEQ ID n° : 1 ou un polypeptide avec au moins 90% d'identité de séquence avec celle-ci et d'un autre récepteur cannabinoïde au moins.

**2.** Procédé selon la revendication 1, dans lequel cet autre récepteur cannabinoïde au moins est choisi dans le groupe composé des CB1, CB1a, CB1b et CB2.

**3.** Procédé, selon la revendication 1 ou la 2, comprenant en outre l'étape consistant à générer un profil du composé à tester.

**4.** Procédé selon l'une quelconque des revendications précédentes, dans lequel le récepteur a la séquence d'acides aminés indiquée à la SEQ ID n° : 1.

**5.** Procédé selon l'une quelconque des revendications précédentes, lequel mesure l'effet du composé de test sur l'interaction de liaison du récepteur et du ligand.

**6.** Procédé selon la revendication 5, dans lequel l'interaction de liaison est suivie d'une mesure directe de la liaison ou de la mesure d'un signal indicatif de l'activation du récepteur ou de l'activité de stimulation cellulaire.

**7.** Procédé selon la revendication 6, dans lequel le signal indicatif de l'activation du récepteur est choisi dans le groupe composé : de l'activité d'une phospholipase C intracellulaire ; de l'activité d'une phospholipase A ; de l'activité d'une adénylyl cyclase ; du taux d'AMPc ; de l'activité d'une MAP kinase ; de la liaison d'un GTP$\gamma$S ; et de la concentration intracellulaire en calcium.

**8.** Procédé selon la revendication 6, dans lequel la détermination de l'interaction de liaison implique la mesure de la quantité de ligand qui forme un complexe avec le récepteur, dans lequel une altération de la quantité dudit complexe formé, en présence dudit composé à tester, identifie ledit composé comme étant un composé qui module la liaison du ligand au récepteur.

**9.** Procédé selon la revendication 8, dans lequel le ligand est choisi dans le groupe composé : de l'anandamide ; de la virohdamine ; de l'éther de noladine ; du 2-arachidonoyl-glycérol ; de la palmitoyléthanolamine ; de l'oléyléthanolamine ; et du D9-tétrahydrocannabinol.

**10.** Procédé selon la revendication 8 ou la 9, dans lequel des complexes récepteur/ligand sont isolés avant de mesurer la quantité de ligand dans lesdits complexes.

**11.** Procédé selon l'une quelconque des revendications 5 à 10, dans lequel le ligand est marqué de manière détectable.

**12.** Procédé selon la revendication 11, dans lequel le ligand est marqué par radioactivité, marqué par fluorescence ou marqué par chimiluminescence.

**13.** Procédé selon la revendication 8, dans lequel le ligand est lié à une enzyme et la mesure est effectuée via un dosage par immuno-absorption enzymatique (ELISA).

**14.** Procédé selon l'une quelconque des revendications précédentes, dans lequel les récepteurs sont exprimés à partir de cellules.

**15.** Procédé selon l'une quelconque des revendications précédentes, dans lequel chaque récepteur du panel est exprimé à partir d'une population différente de cellules.

**16.** Procédé selon les revendications 14 ou 15, dans lequel les cellules sont des cellules de mammifères.

**17.** Procédé selon l'une quelconque des revendications précédentes, dans lequel le récepteur est exprimé à partir d'un gène de récepteur hétérologue.

**18.** Procédé selon l'une quelconque des revendications précédentes, dans lequel l'effet modulatoire du composé à tester vis-à-vis de chaque récepteur est déterminé par :

(i) un contact desdites cellules exprimant le récepteur approprié avec un ligand approprié, en présence ou en l'absence d'un composé à tester ;
(ii) la détermination de l'effet du composé de test sur l'interaction de liaison du récepteur et du ligand ; et
(iii) la comparaison du résultat obtenu au paragraphe (ii) pour chaque récepteur dans le panel.

**19.** Utilisation d'un récepteur GPR55A, ayant la séquence indiquée à la SEQ ID n° : 1 ou un variant de celle-ci avec au moins 90% d'identité de séquence avec la SEQ ID n° : 1, et d'un autre récepteur au moins; choisi dans le groupe composé des CB1, CB1a, CB1b et CB2, dans un crible de panel destiné à tester l'effet modulatoire d'un composé de test vis-à-vis de chacun des récepteurs dans le panel.

**20.** Utilisation d'un récepteur GPR55A, ayant la séquence indiquée à la SEQ ID n° : 1 ou un variant de celle-ci avec au moins 90% d'identité de séquence avec la SEQ ID n° : 1, d'un autre récepteur au moins, choisi dans le groupe composé des CB1, CB1a, CB1b et CB2, et d'un ligand cannabinoïde, destiné à lier chaque récepteur dans un essai sur matrice, pour déterminer l'effet modulatoire d'un composé de test sur la liaison du ligand au récepteur.

**21.** Utilisation selon la revendication 20, dans laquelle le ligand cannabinoïde est choisi dans le groupe composé : de l'anandamide ; de la virohdamine ; de l'éther de noladine ; du 2-arachidonoyl-glycérol ; de la palmitoyléthanolamine ; de l'oléyléthanolamine ; et du D9-tétrahydrocannabinol.

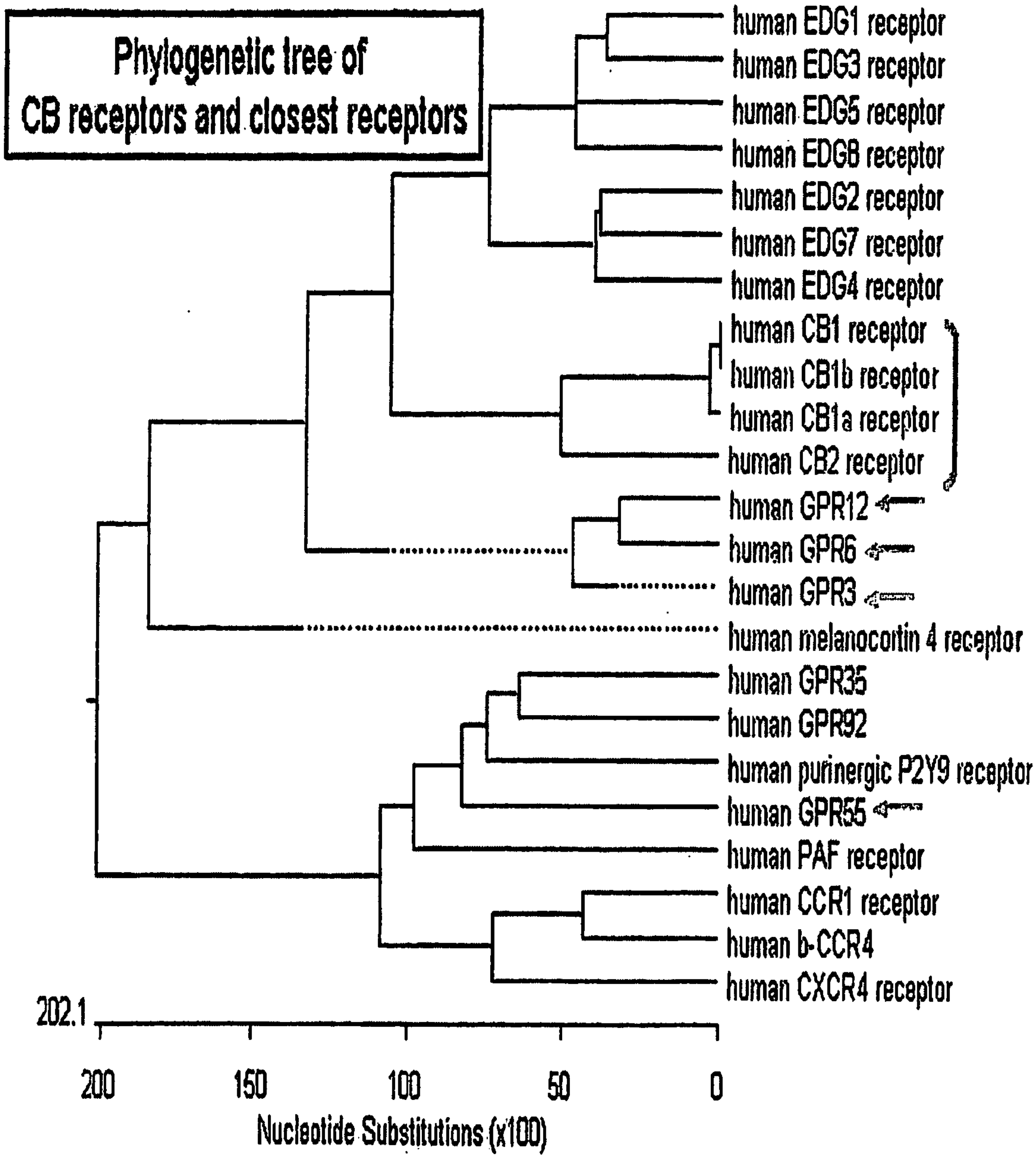
Phylogenetic tree of
CB receptors and closest receptors
human EDG1 receptor
human EDG3 receptor
human EDG5 receptor
human EDG8 receptor
human EDG2 receptor
human EDG7 receptor
human EDG4 receptor
human CB1 receptor
human CB1b receptor
human CB1a receptor
human CB2 receptor
human GPR12
human GPR6
human GPR3
human melanocortin 4 receptor
human GPR35
human GPR92
human purinergic P2Y9 receptor
human GPR55
human PAF receptor
human CCR1 receptor
human b-CCR4
human CXCR4 receptor
202.1
200
150
100
50
0
Nucleotide Substitutions (x100)

**Figure 1**

```
1                                                                              80
                    TM1-----------------TM1     IC1    TM2----------------TM2
MSQQNTSGDCLFDGVNELMKTLQFAVHIPTFVLGLLLNLLAIHGFSTFLKNRWPDYAATSIYMINLAVFDLLLVLSLPFK

81                                                                            160
     EC1         TM3---------------TM3         IC2       TM4-----------------TM4
MVLSQVQSPFPSLCTLVECLYFVSMYGSVFTICFISMDRFLAIRYPLLVSHLRSPRKIFGICCTIWVLVWTGSIPIYSFH
               #10 L                                  SGPPGRSLGSA
                                                         #5 Y

161                                                                           240
      EC2          TM5----------------TM5         IC2        TM6---------------
GKVEKYMCFHNMSDDTWSAKVFFPLEVFGFLLPMGIMGFCCSRSIHILLGRRDHTQDWVQQKACIYSIAASLAVFVVSFL
                           #12 S

241                                                                           319
--------TM6      EC3       TM7-----------------TM7
PVHLGFFLQFLVRNSFIVECRAKQSISFFLQLSMCFSNVNCCLDVFCYYFVIKEFRMNIRAHRPSRVQLVLQDTTISRG
                                     #4 R
```

**Figure 2**

CCPM

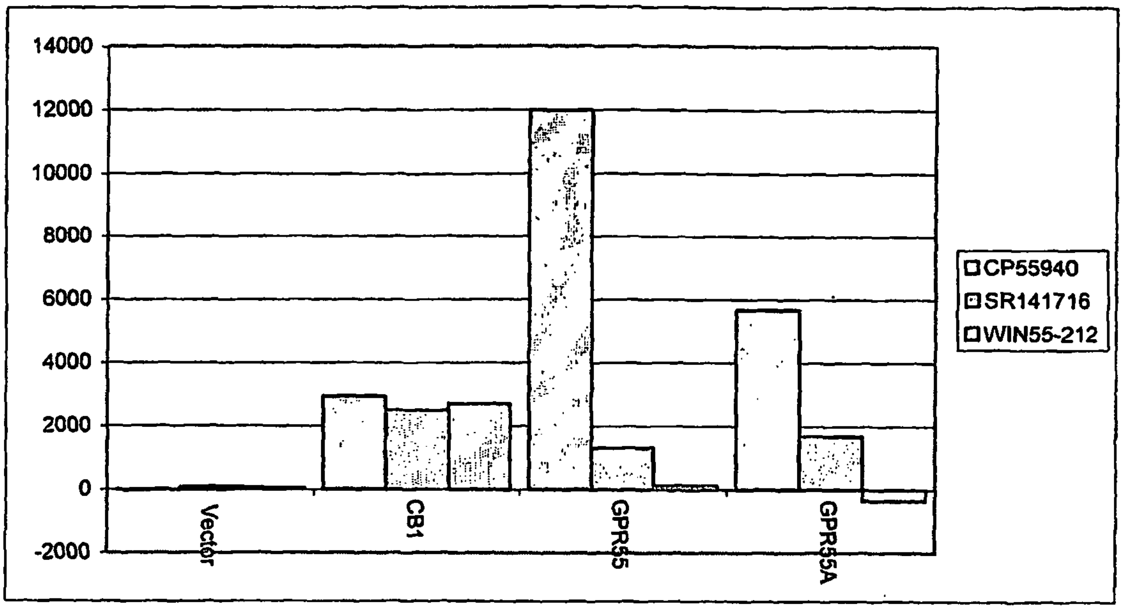


**Figure 3**

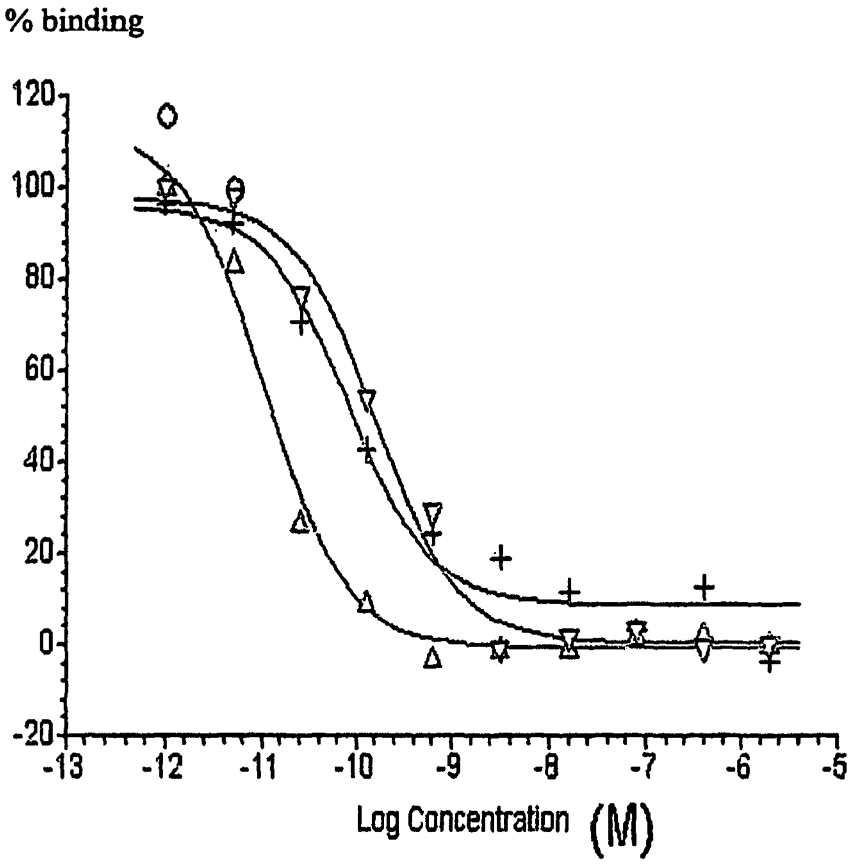
% binding
120
100
80
60
40
20
0
-20
-13
-12
-11
-10
-9
-8
-7
-6
-5
Log Concentration (M)

**Figure 4**

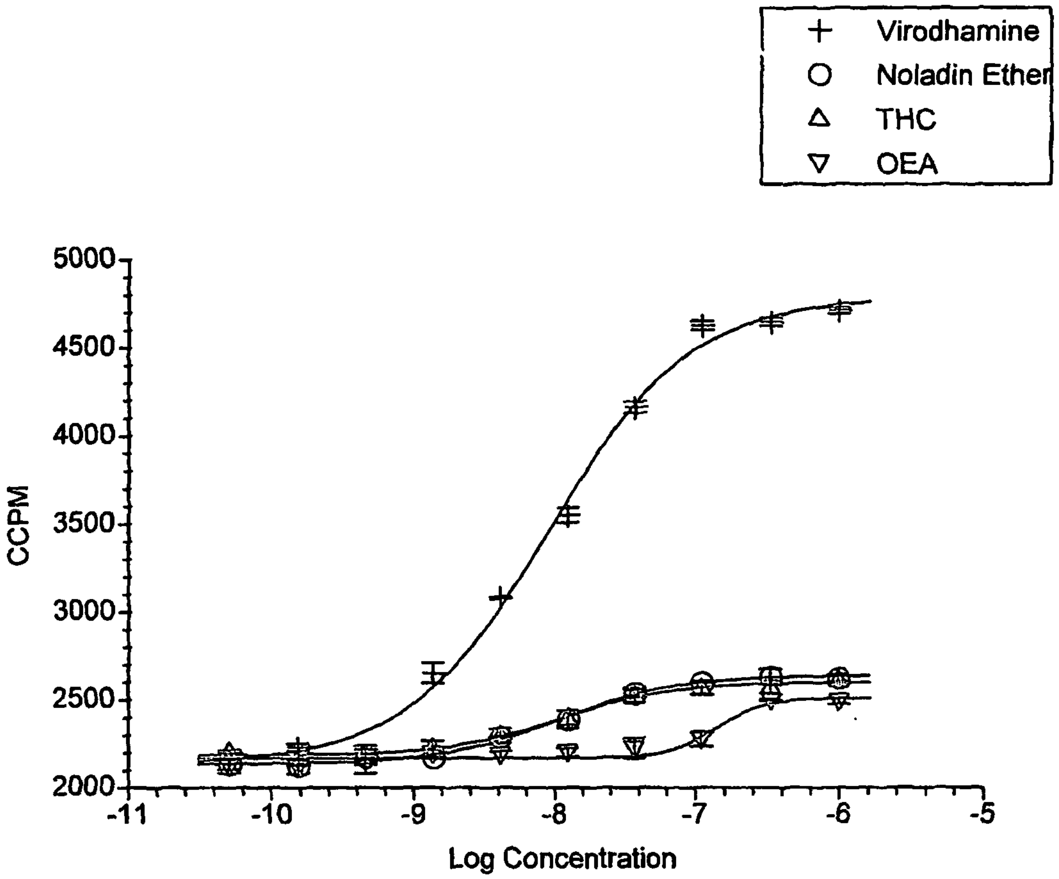
Virodhamine
Noladin Ether
THC
OEA
5000
4500
4000
3500
3000
2500
2000
CCPM
-11
-10
-9
-8
-7
-6
-5
Log Concentration

Figure 5A

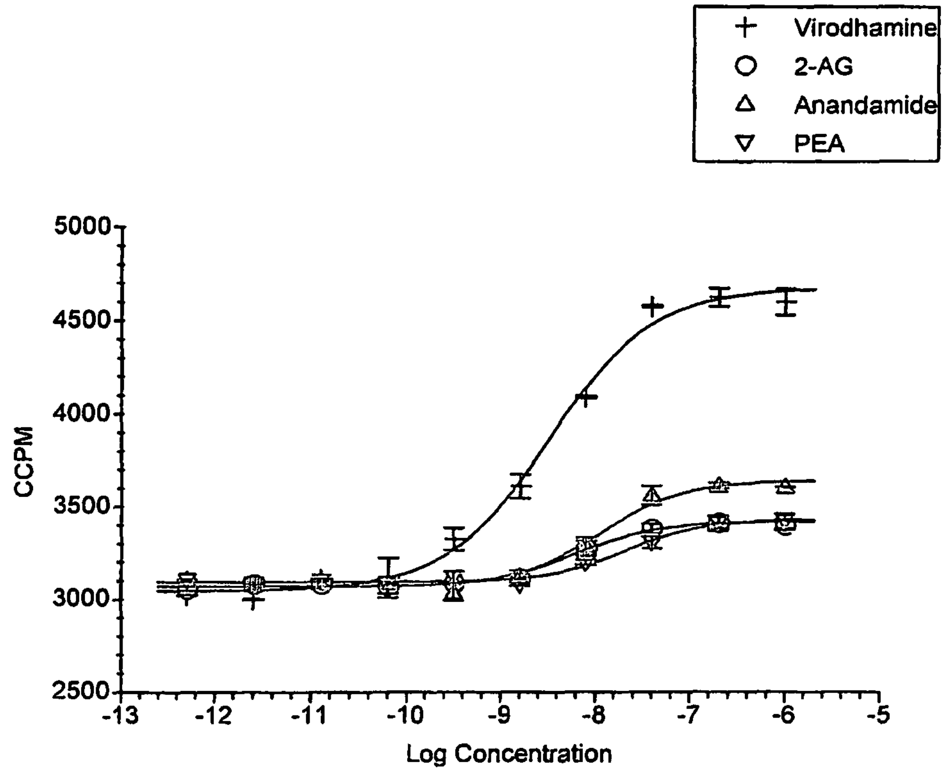
Virodhamine
2-AG
Anandamide
PEA
CCPM
5000
4500
4000
3500
3000
2500
-13
-12
-11
-10
-9
-8
-7
-6
-5
Log Concentration

**Figure 5B**

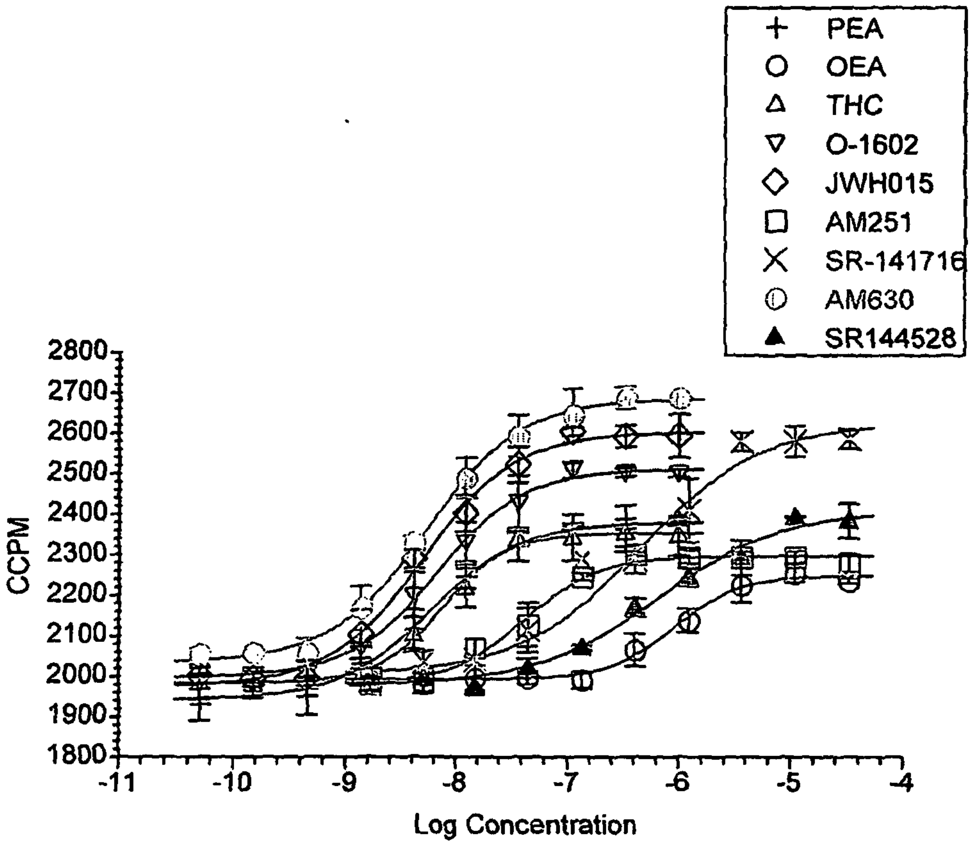
PEA
OEA
THC
O-1602
JWH015
AM251
SR-141716
AM630
SR144528
CCPM
2800
2700
2600
2500
2400
2300
2200
2100
2000
1900
1800
-11
-10
-9
-8
-7
-6
-5
-4
Log Concentration

**Figure 5C**

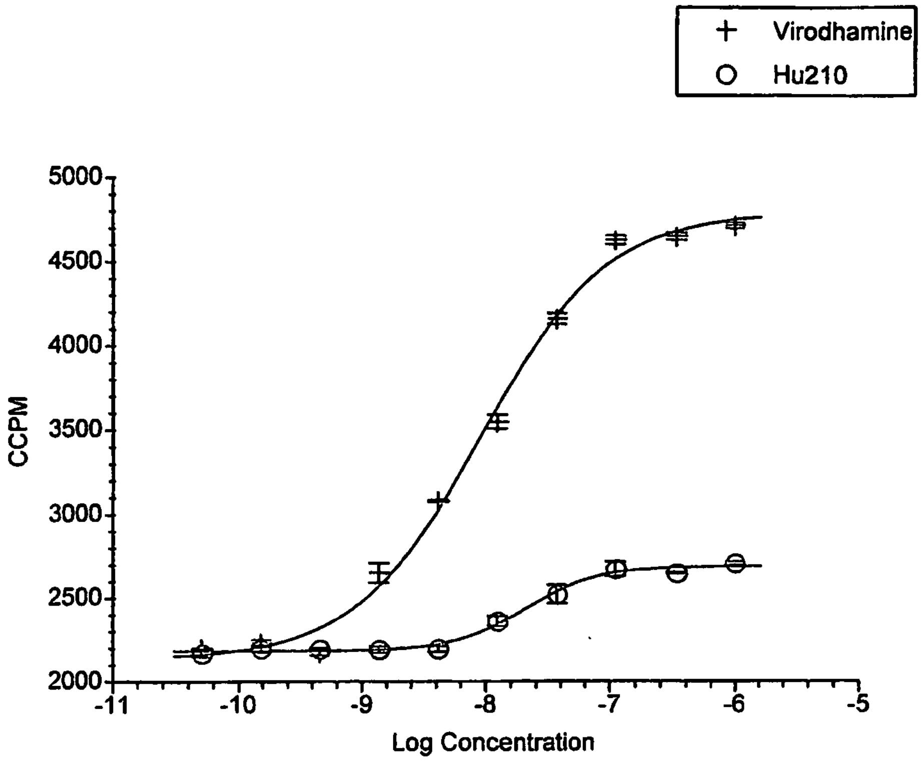
Virodhamine
Hu210
5000
4500
4000
3500
3000
2500
2000
CCPM
-11
-10
-9
-8
-7
-6
-5
Log Concentration

**Figure 5D**

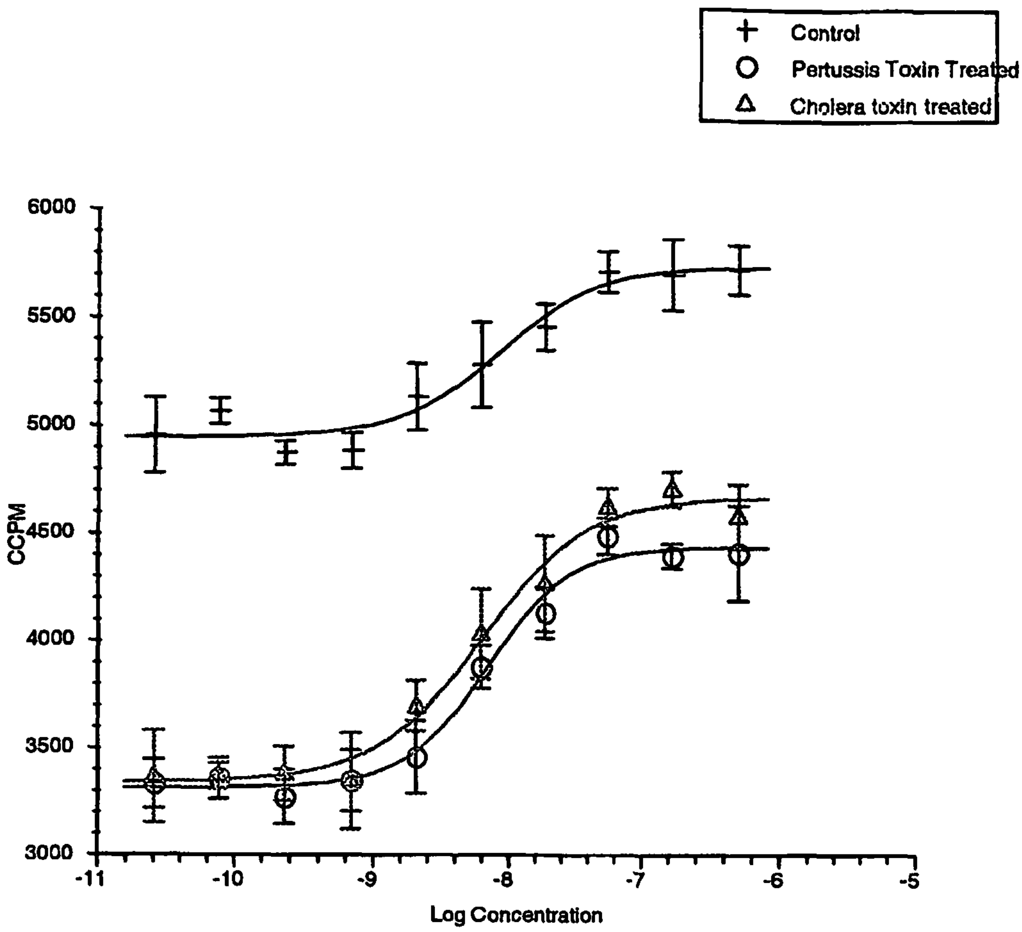
Control
Pertussis Toxin Treated
Cholera toxin treated
6000
5500
5000
4500
4000
3500
3000
CCPM
-11
-10
-9
-8
-7
-6
-5
Log Concentration

**Figure 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0023588 A
- WO 0186305 A
- US 60448665 B


### Non-patent literature cited in the description


- **DEVANE et al.** *Mol. PharmacoL,* 1988, vol. 34, 605-613
- **MATSUDA et al.** *Nature,* 1990, vol. 346, 561-564
- **SHIRE et al.** *J. BioL Chem,* 1995, vol. 270, 3726-3731
- **ISHAC et al.** *Br. J. Pharmacol.,* 1996, vol. 118, 2023-2028
- **MUNRO et al.** *Nature,* 1993, vol. 365, 61-65
- **PERTWEE.** *Pharmacol. Ther.,* 1997, vol. 129, 74
- **ONG ; MACKIE.** *Neuroscience,* 1999, vol. 92, 1177
- **PERTWEE.** *Progr. Neurobiol.,* 2001, vol. 63, 569
- **GALIÈGUE et al.** *Eur. J. Biochem,* 1995, vol. 54, 232
- **FRIDE.** *Prostaglandins Leukot. Essent. Fatty Acids,* 2002, vol. 66, 221-233
- **MACCARRONE et al.** *Prostaglandins Leukot. Essent. Fatty Acids,* 2002, vol. 66, 309-317
- **CAINAZZO.** *Eur. J. Pharmacol,* 2002, vol. 441, 91-97
- **PAROLARO et al.** *Prostaglandins Leukot. Essent. Fatty Acids,* 2002, vol. 66, 319-32
- **VALK et al.** *Blood,* 1997, vol. 90, 1448-1457
- **DEROCQ.** *J. Biol. Chem.,* 2000, vol. 275, 15621-15628
- *Nature,* 1998, vol. 394, 277-281
- **CARON et al.** *Rec. Prog. Horm. Res.,* 1993, vol. 48, 277-290
- **FREEDMAN et al.** *Rec. Prog. Horm. Res.,* 1996, vol. 51, 319-353
- **GUDERMANN et al.** *J. Mol. Med.,* 1995, vol. 73, 51-63
- **SAWZDARGO et al.** *Molecular Brain Research,* 1999, vol. 64, 193-198
- **E. MEYERS ; W. MILLER.** *CABIOS,* 1989, vol. 4, 11-17
- **SAWZDARGO et al.** *Mol. Brain. Res.,* 1999, vol. 64, 193-198
- Molecular Cloning: A Laboratory Manual. Cold Spring Harbor Laboratory Press, 1989
- **HAMER et al.** *J. Mol. Appl. Gen.,* 1982, vol. 1, 273-288
- **YAO et al.** *J. Virol.,* 1995, vol. 69, 6249-6258
- **MCKNIGHT.** *Cell,* 1982, vol. 31, 355-365
- **BENOIST et al.** *Nature,* 1981, vol. 290, 304-310
- **BOSHART et al.** *Cell,* 1985, vol. 41, 521-530
- **WANG et al.** *Proc. Natl. Acad. Sci. U.S.A.,* 1993, vol. 90, 10230-10234
- Current Protocols in Molecular Biology. John Wiley & Sons, 1993, 11.2.1-11.2.19
- Laboratory Techniques in Biochemistry and Molecular Biology. 1978
- **MUNSON.** *Methods Enzymol.,* 1983, vol. 92, 543-577
- **SAWZDARGO et al.** *Mol Brain Res.,* 1999, vol. 64, 193-198
- **MILLIGAN, G.** Signal Transduction: A Practical Approach. Oxford University Press, 1999, 65-67